# EUROPÄISCHE PATENTSCHRIFT

(11) **EP 1 954 180 B1**
(45) Veröffentlichungstag und Bekanntmachung des Hinweises auf die Patenterteilung: **11.11.2009**
(21) Anmeldenummer: 06830295.9
(22) Anmeldetag: 01.12.2006
(51) Int. Cl.: A61B 5/00, G09G 3/32

(54) **ANALYSESYSTEM MIT ORGANISCHER LEUCHTDIODENANZEIGE**
ANALYSIS SYSTEM HAVING AN ORGANIC LIGHT-EMITTING DIODE DISPLAY
SYSTEME D'ANALYSE AVEC AFFICHAGE ORGANIQUE PAR DIODES ELECTROLUMINESCENTES

(30) Priorität: 02.12.2005 EP 05026289
(43) Veröffentlichungstag der Anmeldung: 13.08.2008
(73) Patentinhaber: F.HOFFMANN-LA ROCHE AG, 4070 Basel (CH); Roche Diagnostics GmbH, 68305 Mannheim (DE)
(72) Erfinder: KALVERAM, Stefan, 68519 Viernheim (DE); HECK, Wolfgang, 68526 Ladenburg (DE); MENKE, Andreas, 68305 Mannheim (DE); SCHMID, Wilfried, 68165 Mannheim (DE); ZIEGLER, Friedrich, 70499 Stuttgart (DE); SCHMELZEISEN-REDEKER, Günther, 64653 Lorsch (DE)
(74) Vertreter: Hörschler, Wolfram Johannes
(86) Internationale Anmeldenummer: PCT/EP2006/069229
(87) Internationale Veröffentlichungsnummer: WO 2007/063135

(56) Entgegenhaltungen:
- DE-A1- 10 253 154
- DE-A1- 10 341 093
- DE-U1-202005 012 358
- US-A- 6 144 162
- US-A1- 2003 004 403
- US-A1- 2005 007 392
- US-A1- 2005 113 655
- US-A1- 2005 143 671

## Beschreibung

Die Erfindung betrifft ein portables medizinisches System, welches insbesondere für Analyse- und/oder Medikationszwecke eingesetzt werden kann. Beispielsweise werden derartige portable medizinische Systeme im Rahmen von "Homecare-Systemen", z. B. bei der Blutglucoseüberwachung oder einer Selbstmedikation, beispielsweise mittels Insulinpumpen, eingesetzt.

### Stand der Technik

Die Bestimmung von Blutglucosekonzentrationen sowie eine entsprechende Medikation ist für Diabetiker ein essentieller Bestandteil des Tagesablaufs. Dabei muss die Blutglucosekonzentration schnell und einfach mehrmals am Tag (typischerweise zwei- bis siebenmal) bestimmt werden, um gegebenenfalls entsprechende medizinische Maßnahmen ergreifen zu können. In vielen Fällen erfolgt dabei eine Medikation mittels automatischer Systeme, insbesondere so genannter Insulinpumpen.

Um den Tagesablauf des Diabetikers nicht mehr als nötig einzuschränken, werden häufig entsprechende mobile Geräte eingesetzt, welche einfach zu transportieren und zu handhaben sein sollten, so dass eine Messung der Blutglucosekonzentration beispielsweise am Arbeitsplatz oder auch in der Freizeit problemlos erfolgen kann. Derzeit sind verschiedene mobile Geräte am Markt, welche teilweise nach unterschiedlichen Messmethoden und unter Verwendung unterschiedlicher Diagnoseverfahren funktionieren. Ein erstes Messverfahren beruht beispielsweise auf einer elektrochemischen Messmethode, wobei eine Blutprobe auf eine mit Enzymen und Mediatoren beschichtete Elektrode appliziert wird. Entsprechende Teststreifen für derartige elektrochemische Messverfahren sind beispielsweise in US 5,286,362 beschrieben. Andere bekannte Messmethoden verwenden optische Messverfahren, welche beispielsweise darauf beruhen, dass die zu detektierende Substanz mit bestimmten Nachweisreagenzien reagieren kann, wobei eine Farbänderung des Reaktionsgemischs eintritt. Systeme zum Nachweis derartiger Farbreaktionen und somit zum Nachweis der entsprechenden Analyten sind beispielsweise aus CA 2,050,677 bekannt.

Es hat sich gezeigt, dass aufgrund allgemeiner altersbedingter Zunahme von Erkrankungen Diabetes häufig bei älteren Menschen auftritt. Gerade bei älteren Menschen ist jedoch vielfach die Schkraft, insbesondere unter schlechten Lichtverhältnissen, altersbedingt eingeschränkt. Zudem sind Augenschädigungen eine häufige Form der typischen Folgeschäden des Diabetes Mellitus. Daher benötigen insbesondere Diabetiker gut ablesbare Anzeigeelemente in den portablen, zur Selbstkontrolle verwendeten Messgeräten. Ähnliche Probleme treten auch bei anderen Arten von Erkrankungen auf, bei welchen portable Geräte im Rahmen eines Homecare-Konzeptes verwendet werden.

Die derzeit auf dem Markt befindlichen portablen medizinischen Systeme, insbesondere portable Glucose-Messgeräte, setzen jedoch typischerweise Flüssigkristallanzeigen (Liquid Crystal Displays, LCDs) als Anzeigeinstrumente für Glucose-Messwerte, Warnungen und Hinweise, Datum, Uhrzeit etc. ein. Dabei kommen sowohl segmentierte LCDs als auch so genannte Matrix-LCDs zum Einsatz. Aufgrund ihrer geringen Kosten und einer einfacheren Ansteuerung werden überwiegend segmentierte LCDs eingesetzt. Matrix-LCDs sind in wenigen Glucose-Messgeräten enthalten, wobei es sich bei diesen Geräten häufig um hochwertige Geräte mit umfangreichen Datenmanagementfunktionen handelt.

Flüssigkristallanzeigen weisen jedoch hinsichtlich ihrer Ablesbarkeit einige Nachteile auf. So sind Flüssigkristallanzeigen insbesondere nicht selbst leuchtend. Die Flüssigkristallelemente wirken vielmehr lediglich als "Schalter" zum Ein- und Ausschalten einer lokalen Transparenz. Die dargestellten Zeichen werden somit dadurch sichtbar, dass transmittiertes Licht an bestimmten Punkten der Anzeige oder in bestimmten Bereichen der Anzeige blockiert bzw. hindurchgelassen wird. Die Bereitstellung des Lichts muss jedoch durch andere Mittel als die Flüssigkristallanzeige selbst erfolgen. Zum einen kann dies dadurch geschehen, dass Umgebungslicht an einer reflektierenden Fläche hinter der Flüssigkristallanzeige reflektiert und durch die Flüssigkristallanzeige transmittiert wird. In diesem Fall hängt jedoch die Ablesbarkeit der Flüssigkristallanzeige stark von der Beleuchtungsstärke des Umgebungslichts ab. In dunkler oder lichtschwacher Umgebung können Flüssigkristallanzeigen nur schlecht oder gar nicht abgelesen werden.

Diese Abhängigkeit der Flüssigkristallanzeigen vom Umgebungslicht lässt sich dadurch reduzieren, dass die Flüssigkristallanzeigen von hinten oder seitlich (zum Beispiel mittels Leuchtfolien oder Leuchtdioden) durchleuchtet werden (Hintergrundbeleuchtung "Backlight Display"). Ein Nachteil dieser Technik besteht jedoch darin, dass der Kontrast der Anzeige unter guten Lichtverhältnissen beeinträchtigt wird. Dieser Kontrast kann nicht gleichzeitig für eine Darstellung ohne und mit Hintergrundbeleuchtung optimiert werden, weshalb eine fallweise Benutzung einer Hintergrundbeleuchtung immer zu einem Kompromiss im Kontrast des Anzeigeelements führt. Zudem verbraucht eine Hintergrundbeleuchtung vergleichsweise viel elektrische Energie, was zu einer reduzierten Lebensdauer von Batterien in dem Messgerät führen kann. Insbesondere bei portablen Messgeräten, beispielsweise portablen Glucose-Messgeräten, macht sich diese reduzierte Lebensdauer nachteilig bemerkbar.

Ein weiterer Nachteil der Verwendung von Flüssigkristallanzeigen ist, dass die Ablesbarkeit der Flüssigkristallanzeige stark vom Ablesewinkel (typischerweise definiert als der Winkel zwischen einer Normalen zum Anzeigenelement und der Blickrichtung eines Beobachters) abhängig ist. Dieser Effekt trifft sowohl mit als auch ohne zusätzliche Hintergrundbeleuchtung auf. Dadurch ist die Benutzungsfreiheit des Glucose-Messgeräts durch den Diabetiker stark eingeschränkt. Dies macht sich insofern besonders nachteilhaft bemerkbar, da viele Diabetiker das Glucose-Messgerät verwenden, indem sie es zur Messung und Bedienung auf eine Tischplatte legen. Hier können situationsbedingt Ablesewinkel auftreten, bei denen das Ablesen des Displays beeinträchtigt oder sogar unmöglich ist.

Neben Flüssigkristallanzeigen sind eine Reihe weiterer Displaytechnologien bekannt. So ist aus anderen Bereichen der Technik die Technologie der organischen Leuchtdioden (OLEDs) bekannt, welche in verschiedenen technischen Modifikationen eingesetzt wird. Bei organischen Leuchtdioden werden dünne organische Schichten (eine oder mehrere organische Schichten mit einer Gesamtdicke von typischerweise zwischen 50 und 300 nm) zwischen zwei Elektroden eingebettet. Wird ein elektrischer Strom durch die organischen Schichten geleitet, so findet, ähnlich zu anorganischen Halbleitern, in den organischen Schichten eine Rekombination von "Elektronen" und "Löchern" (bzw. deren organischen Pendants) statt. Bei dieser Rekombination werden Photonen emittiert. Dieser Effekt wird als organische Elektrolumineszenz bezeichnet.

Üblicherweise werden organische Leuchtdioden als Dünnschichtsysteme auf einem transparenten Substrat, beispielsweise einem Glas- oder Kunststoffsubstrat aufgebaut. Dabei werden üblicherweise nacheinander Elektroden- und organische Schichten aufgebaut, bis der oben beschriebene "Sandwich-Aufbau" entsteht. Üblicherweise wird dabei als erste Elektrodenschicht (zum Beispiel Anodenschicht) eine transparente Elektrodenschicht verwendet, beispielsweise Indium-Zinn-Oxid. Als Gegenelektrode (üblicherweise Kathode) wird beispielsweise eine Metallschicht, zum Beispiel Kalzium oder Magnesium, verwendet. Anschließend wird der "Sandwich-Aufbau" entsprechend verkapselt, um den Aufbau gegen Einfluss von Luftfeuchtigkeit und Sauerstoff zu schützen. Neben diesem beschriebenen Standardaufbau sind auch andere Aufbauten bekannt, beispielsweise Aufbauten mit mehreren, aufeinander gestapelten OLEDs oder Aufbauten, bei welchen nicht durch das Glassubstrat emittiert wird, sondern durch eine transparente Metallelektrodenschicht. Weiterhin existieren verschiedene Technologien, welche sich bezüglich der eingesetzten organischen Materialien unterscheiden. So existieren Technologien, bei welchen die Materialien sich aus (in der Regel aufgedampften) monomolekularen Substanzen zusammensetzen. Andere Technologien verwenden als organische Materialien Polymere, welche in der Regel nasschemisch aufgebracht werden. Auch hybride Technologien sind dem Fachmann bekannt.

Organische Leuchtdioden werden mittlerweile in verschiedenen Bereichen der Technik eingesetzt. Ein Beispiel hierbei sind Mobiltelefone, Mischpulte im Audiobereich, Displays für digitale Kameras, sowie MP3-Player oder Multimediaplayer. Auch im Bereich der medizinischen Technik finden sich Anwendungsbeispiele. So beschreiben beispielsweise WO 2004/048881 A2 und US 2003/0035109 A1 Systeme, bei welchen organische Leuchtdioden als Lichtquellen eingesetzt werden. WO 2004/048881 A2 offenbart eine Messeinrichtung zur optischen Untersuchung eines diagnostischen Testelements mit einer Lichtquelle, einem Photodetektor und einer Vorrichtung zum Positionieren des Testelements. Die Lichtquelle weist eine oder mehrere organische Leuchtdioden auf. US 2003/0035109 A1 offenbart ein Gerät zur Detektion organischer Moleküle, insbesondere Biomoleküle oder Polymere, wobei zu Beleuchtungszwecken unter anderem auch der Einsatz einer O-LED vorgeschlagen wird.

Neben dem Einsatz als Beleuchtungsmittel sind auch Anwendungen von OLEDs als Anzeigeelemente in der medizinischen Technik bekannt. So offenbart US 2005/0015115 A1 ein Erste-Hilfe-System, welches ein Ausgabegerät aufweist. Dabei wird darauf hingewiesen, dass das Ausgabegerät auch ein OLED-Display aufweisen kann. Auch US 6,579,237 B1 offenbart ein medizinisches System mit einem OLED-Display. Dabei handelt es sich um ein diagnostisches Ultraschall-Bildgebungssystem, welches ein OLED-Display zur Darstellung der Ultraschalldaten aufweist.

Bei den aus dem Stand der Technik bekannten medizinischen Systemen, welche OLED-Displays einsetzen, handelt es sich somit überwiegend um stationäre Systeme von erheblicher Größe, welche von einem Patienten nicht ohne weiteres am Körper getragen werden können. Weiterhin offenbart DE 102 53 154 A1 ein Messgerät zur Bestimmung eines Analyten in einer Flüssigkeitsprobe, welches ein Testelement mit einem Testfeld sowie einen Detektor aufweist. Die Besonderheit dieser Vorrichtung besteht darin, dass elektrische Gerätekomponenten zum Einsatz kommen, welche zumindest teilweise auf der Basis von Polymerelektronik ausgebildet sind. Der Nachteil derartiger Messgeräte auf der Basis von Polymerelektronik besteht jedoch darin, dass Polymerelektronik, insbesondere Transistoren auf organischer Basis, nach dem Stand der Technik noch vergleichsweise störanFällig ist und nur Ausgestaltungen mit vergleichsweise geringer elektronischer Funktionalität ermöglichen. Ein tragbares Pulsoxymeter mit optionaler OLED-Anzeige wird in US 2005/0113655 beschrieben.

US 2003/0004403 A1 beschreibt Verfahren und Vorrichtungen für eine kontinuierliche Fernüberwachung physiologisch relevanter Zustände. Dabei werden physiologische Abweichungen vom Normalzustand automatisch detektiert und ein Patient entsprechend gewarnt. Dabei wird ein so genannter Bio-Interface-Kopf mit Sensoren durch ein Kontroll- und Kommunikationsmodul angesteuert, und dabei gewonnene Daten werden zu einer Datensammeleinheit übermittelt. Eine Leistungs-Regelungs-Funktion im CCM sorgt dabei für eine Optimierung der Leistungsversorgung des Bio-Interface-Kopfes mit elektrischer Energie. Weiterhin wird in US 2003/0004403 A1 auch die Möglichkeit offenbart, das Kontroll- und Kommunikationsmodul mit OLED-Displays auszustatten.

Eine Problematik der Verwendung von OLED-Displays, welche aus anderen Bereichen der Technik bekannt ist, besteht häufig darin, dass die eingesetzten Displays eine vergleichsweise geringe Lebensdauer aufweisen und zu hoher Störanfälligkeit neigen. Dies liegt insbesondere darin, dass die eingesetzten organischen Materialien mit der Zeit degradieren. Weiterhin gestaltet sich die Qualitätskontrolle häufig schwierig, und beispielsweise die eingesetzten Elektrodenmaterialien (zum Beispiel reaktive Metalle wie Kalzium oder Magnesium) neigen zu Oxidationseffekten. Diese Effekte führen dazu, das einzelne Bildpunkte, einzelne Zeilen oder Spalten, sowie teilweise ganze Displays langsam oder unvorhergesehen plötzlich ausfallen. Die beispielsweise in US 2003/0004403 A1 vorhandene und auch in anderen medizinischen Geräten eingesetzte Leistungs-Regelungs-Funktion ist in der Regel nicht in der Lage, derartige minimale Effekte, wie sie beispielsweise durch Ausfall einzelner Zeilen oder Spalten entstehen, zu detektieren und entsprechend zu reagieren.

Ein derartiger Ausfall ist jedoch gerade bei medizinischen Geräten, insbesondere medizinischen Geräten welche im privaten Bereich zur Selbstkontrolle und/oder Selbstmedikation eingesetzt werden, oft mit fatalen Folgen verbunden. So kann es sein, dass insbesondere ältere Patienten auftretende Fehler nicht wahrnehmen oder, selbst wenn die Fehler wahrgenommen werden, nicht sachgemäß auf diese Fehler reagieren. Dies kann beispielsweise zu einer fehlerhaften Medikation - mit den bekannten gravierenden Folgen - führen. Insbesondere haben sich hier so genannte segmentierte Displays, beispielsweise 7-Segment-Anzeigen, als nachteilig erwiesen, da hier durch unbemerkten Ausfall einzelner Segmente leicht eine Verfälschung von angezeigten Werten auftreten kann. So kann beispielsweise aus einer Anzeige "7" leicht die Anzeige "1" werden, wenn das oberste waagerechte Segment ausfällt. Ein derartiger Defekt kann bei Displays im medizinischen Bereich fatale Folgen mit sich bringen.

### Aufgabe der Erfindung

Es ist daher eine Aufgabe der vorliegenden Erfindung, ein portables medizinisches System anzugeben, welches hohe Benutzerfreundlichkeit hinsichtlich der oben beschriebenen Anzeigeeigenschaften aufweiset sowie eine hohe Handhabungssicherheit unter weitgehender oder vollständiger Vermeidung der beschriebenen Nachteile des Standes der Technik. Auftretende Fehler sollten möglichst umgehend erkannt werden, um entsprechende Maßnahmen ergreifen zu können.

### Darstellung der Erfindung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Es wird ein portables medizinisches System vorgeschlagen, welches insbesondere für Analyse- und/oder Medikationszwecke einsetzbar ist. Unter "portabel" soll dabei verstanden werden, dass das medizinische System im Rahmen eines "Homecare-Systems" vom Patienten mitgeführt werden kann, um beispielsweise zu Hause, am Arbeitsplatz oder in der Freizeit eine medizinische Grundversorgung sicherzustellen. Zu diesem Zweck soll das portable medizinische System klein ausgebildet sein, soll beispielsweise Abmessungen ähnlich den Abmessungen eines typischen Mobiltelefons aufweisen und soll vorzugsweise nur eine geringe Anfälligkeit gegenüber Störeinflüssen wie mechanischen Erschütterungen und/oder Feuchtigkeit haben.

Insbesondere soll sich die Erfindung beziehen auf ein medizinisches System, welches ein Analysesystem zum Nachweis mindestens eines Analyten in einer Probe aufweist. Derartige Systeme können beispielsweise die oben beschriebenen Systeme umfassen, welche auf entsprechenden Teststreifen und Geräten zur Auswertung derartiger Teststreifen beruhen. Insbesondere kann es sich dabei um Geräte zum Nachweis von Blutglucose und/oder Cholesterol und/oder einer Koagulation handeln. Auch andere Analyten können nachweisbar sein, beispielsweise ein Lactatgehalt. Insbesondere kann das Analysesystem dabei, wie oben beschrieben, mindestens ein Reagenz einsetzen, welcher mit dem nachzuweisenden Analyten reagiert und dabei eine elektrochemische Veränderung des Reaktionsgemischs und/oder eine Farbreaktion auslöst.

Weiterhin kann das medizinische System alternativ oder zusätzlich auch eine Medikationsvorrichtung, insbesondere eine Medikationspumpe, umfassen. Insbesondere kann es sich dabei um eine Insulinpumpe für eine selbstständige Medikation mit Insulin handeln, beispielsweise eine Insulinpumpe, welche in regelmäßigen Abständen eine vorgegebene Insulindosis abgibt oder welche eine Dosiermenge entsprechend eines Eingangssignals einstellt. Alternativ oder zusätzlich zu einer Analysevornchtung oder Medikationsvorrichtung kann das portable medizinische System auch eine andere Art medizinischer Überwachungsvorrichtung, z.B. eine künstliche Bauchspeicheldrüse (Artificial Pancreas) und/oder ein System zur kontinuierlichen Überwachung (Continuous Monitoring) aufweisen. Derartige Vorrichtungen sind dem Fachmann in verschiedenen Ausgestaltungen bekannt und werden nachstehend noch eingehender erläutert.

Ein Grundgedanke der vorliegenden Erfindung besteht darin, dass das portable medizinische System mindestens ein Anzeigenelement aufweist, welches mindestens eine organische Leuchtdiodenanzeige umfasst. Dabei kann es sich besonders vorteilhafterweise um ein Matrix-Display, beispielsweise ein organisches Passiv-Matrix-Display handeln. Matrix-Displays weisen, im Gegensatz beispielsweise zu segmentierten Displays, die oben beschriebenen Nachteile nicht auf, dass Ausfälle oft nur schwer zu erkennen sind. Bei Matrix-Displays treten Ausfälle und Defekte typischerweise als (in der Regel unschädliche) Punktdefekte oder als leicht zu erkennende Zeilen- und/oder Spaltenausfälle auf. Die Möglichkeit einer falschen Ablesung ist somit weiter stark reduziert.

Neben dem mindestens einen Anzeigenelement können zusätzlich auch weitere Anzeigenelemente unter Verwendung anderer Technologien eingesetzt werden, beispielsweise Flüssigkristallanzeigen oder anorganische Leuchtdiodenanzeigen.

Grundsätzlich sind für die organische Leuchtdiodenanzeige alle aus dem Stand der Technik bekannten, insbesondere die oben beschriebenen, OLED-Techniken einsetzbar. Insbesondere lassen sich OLEDs einsetzen, welche auf der Basis von monomolekularen organischen Substanzen aufgebaut sind sowie Displays, welche Polymere umfassen. Auch Mischtechnologien (Hybridtechnologien) sind denkbar. Weiterhin sind nicht nur Matrix-Displays einsetzbar, sondern es können beispielsweise auch Segment-Displays eingesetzt werden, beispielsweise 7-Segment-Anzeigen, oder einfache Symbol-Displays, beispielsweise Displays mit Batteriestandsanzeigen oder Warnanzeigen. Auch Aktiv-Matrixgesteuerte Displays sind prinzipiell möglich, wobei in diesem Falle jedoch der Kostenaufwand erhöht ist (was insbesondere bei hochwertigen Geräten jedoch einen tragbaren Nachteil darstellt).

Der Vorteil der Verwendung von organischen Leuchtdiodenanzeigen gegenüber herkömmlichen Flüssigkristallanzeigen ist vielfältig. Insbesondere lassen sich erheblich höhere Kontraste erzielen, wodurch die Ablesbarkeit selbst bei kleinen Schriftgrößen erheblich verbessert wird. Ein weiterer Vorteil besteht darin, dass es sich bei organischen Leuchtdiodenanzeigen um selbst-leuchtende Anzeigeelemente handelt, welche auch im Dunkeln bzw. bei schlechten Lichtverhältnissen sehr gut ablesbar sind. Auf Hintergrundbeleuchtungen kann verzichtet werden, was den Energieverbrauch gegenüber herkömmlichen Flüssigkristallanzeigen deutlich verringert. Dadurch ergeben sich erheblich längere Batterielebensdauern, was insbesondere bei Glucose-Messgeräten mit Motoren und anderen "Energiefressern" vorteilhaft ist.

Ein weiterer erheblicher Vorteil der Verwendung von organischen Leuchtdiodenanzeigen gegenüber herkömmlichen LCDs besteht im großen möglichen Ablesewinkel. So umfasst der Ablesewinkel einen Bereich von nahezu 180° (das heißt von nahezu -90° bis nahezu +90°). Insbesondere bei der oben beschriebenen Verwendung, bei welcher ein Blutglucose-Messgerät vor dem Patienten auf eine Tischplatte abgelegt wird, stellt dies einen erheblichen Vorteil dar.

Ein weiterer Vorteil der organischen Leuchtdiodenanzeigen besteht in der möglichen dünnen und flachen Bauweise der Anzeigen. So lassen sich Anzeigen mit einer Gesamtdicke von unter einem Millimeter herstellen. Dadurch kann nicht nur Bauraum eingespart und somit das Gesamtvolumen reduziert werden, was für portable Geräte einen erheblichen Benutzervorteil darstellt. Auch lässt sich das Gewicht der portablen medizinischen Systeme auf diese Weise erheblich reduzieren, so dass die Benutzerfreundlichkeit stark erhöht wird. Zudem sind organische Leuchtdiodenanzeigen kostengünstig herstellbar, so dass auch low-cost-Geräte mit dieser Technologie ausgestattet werden können.

Ein weiterer Vorteil besteht darin, dass sich organischen Leuchtdiodenanzeigen gegenüber herkömmlichen LCDs, insbesondere gegenüber Passiv-Matrix-LCDs, erheblich flexibler einsetzen lassen. Ein Punkt hierbei besteht darin, dass die organischen Leuchtdiodenanzeigen auch in einer Passiv-Matrix-Steuerung hervorragende Schaltzeiten, das heißt insbesondere Schaltzeiten weit unterhalb einer Millisekunde, aufweisen, wodurch derartige Passiv-Matrix-Displays bereits als solche videotauglich sind, somit lassen sich auch sehr schnelle Bildfolgen darstellen, beispielsweise schnell veränderliche Messwerte oder animierte Graphiken. Bei herkömmlichen Flüssigkristallanzeigen ist dies nur unter Verwendung zusätzlicher Technologien, insbesondere unter Verwendung von Transistorschaltungen zur Ansteuerung (Aktiv-Matrix-Schaltungen) möglich. Weiterhin lassen sich insbesondere organische Leuchtdiodenanzeigen in Form von Matrix-Displays sehr flexibel gestalten und ermöglichen eine flexible Darstellung des Display-Inhalts und somit eine wesentlich bessere Benutzerführung des Diabetikers als mittels eines Segment-Displays. Dies macht sich nicht nur beim Messen der Blutglucosekonzentration, sondern auch bei anderen Einstellungen, wie zum Beispiel Setup-Einstellungen, sowie bei Datenmanagementfunktionen, Warnungen, Hinweisen, Darstellungen von (Kurz-)Bedienungsanleitungen etc. positiv bemerkbar.

Erfindungsgemäß wird bei dem portablen medizinischen System die Tatsache ausgenutzt, dass OLED-Anzeigen stromgetrieben sind (das heißt die Helligkeit ist typischerweise proportional zum durch die OLED geleiteten Strom), wohingegen typische Flüssigkristallanzeigen spannungsgesteuert sind. Dementsprechend ist das vorgeschlagene portable medizinische System mit einer Überwachungsvorrichtung ausgestattet. Diese Überwachungsvorrichtung soll eine Vorrichtung zur Erfassung eines Treiberstromes durch die mindestens eine organische Leuchtdiodenanzeige umfassen. Dabei ist eine Vorrichtung zum Vergleich des Treiberstromes mit einem vorgegebenen Sollwert vorgesehen.

Auf diese Weise lässt sich eine so genannte "Failsafe -Funktion" realisieren, indem beispielsweise Strom-Ausgangsstufen von Treiber-ICs zum Treiben der OLEDs überwacht werden, dahingehend, ob ein eingestellter Strom auch tatsächlich fließt. Im Falle einer Abweichung zwischen einem Soll- und einem Ist-Wei-t kann auf diese Weise beispielsweise auf einen Fehler rückgeschlossen werden. So ist es teilweise sogar möglich, auf die Art des Fehlers zurückzuschließen, beispielsweise einen Fehler in einem bestimmten Bildpunkt (Pixel) und/oder in einer bestimmten Zeile und/oder in einer bestimmten Spalte. Diese Fehler können beispielsweise an das portable medizinische System, beispielsweise einen zentralen Computer, zurückgemeldet werden.

Die Überwachungsvorrichtung weist weiterhin beispielsweise mindestens eine Wamvorrichtung zur Erzeugung einer Warnung für einen Benutzer auf, welche bei Abweichung des Treiberstroms von dem vorgegebenen Sollwert um mehr als einen vorgegebenen Betrag eine Warnung an einen Benutzer ausgibt. Auf diese Weise können beispielsweise auf dem mindestens einen Anzeigeelement (das heißt zum Beispiel auf der organischen Leuchtdiodenanzeige selbst oder einer separaten Anzeige, beispielsweise einem Warnsymbol) Warnungen und entsprechende Anweisungen ausgegeben werden. Auf diese Weise kann verhindert werden, dass Patienten mit fehlerhaften Messwerten oder Ausgabewerten, die auf dem mindestens einen Anzeigeelement dargestellt sind, arbeiten und beispielsweise dementsprechend eine Medikation berechnen. So kann zum Beispiel die Warnung ausgegeben werden, dass das portable medizinische System gewartet werden muss und/oder nicht mehr verwendet werden darf. Die oben beschriebenen Gefahren einer Falschanzeige mit der Gefahr einer fatalen fehlerhaften Medikation sind somit nahezu ausgeschlossen. Dies ist gerade für Anwendungen im medizinischen Bereich, in dem es auf absolute Zuverlässigkeit der eingesetzten Geräte ankommt, ein entscheidender Vorteil.

Die Möglichkeit, direkt an einer Schnittstelle hin zum eigentlichen Display zu messen und dementsprechend einen Fehler zu detektieren, ist durch die strombetriebene Funktionsweise der organischen Leuchtdiodenanzeige im Gegensatz zum spannungsgeschalteten Flüssigkristallanzeigeelement stark vereinfacht. Hierbei machen sich insbesondere bei üblichen Flüssigkristallanzeigen Fertigungstoleranzen bemerkbar, welche üblicherweise eine vergleichbare Messung an einer Schnittstelle hin zum Display nur schwer realisierbar machen.

Das erfindungsgemäße portable medizinische System lässt sich zusätzlich auf verschiedene Weise vorteilhaft weiterbilden. So kann beispielsweise die mindestens eine organische Leuchtdiodenanzeige mindestens ein flexibles Anzeigenelement aufweisen. Dabei kann es sich insbesondere um ein Anzeigenelement mit einem transparenten flexiblen Substrat, insbesondere einem flexiblen Glas- und/oder Kunststoffsubstrat, handeln. Unter "flexibel" soll dabei im Gegensatz zu starren Displays verstanden werden, dass zumindest eine geringfügige Verbiegung durch händische Krafteinwirkung herbeigeführt werden kann. Beispielsweise kann die organische Leuchtdiodenanzeige mit einem flexiblen transparenten Kunststoff- oder Glassubstrat ausgestattet werden, wobei beispielsweise Glassubstrate mit einer Dicke von weniger als 200 µm, vorzugsweise weniger als 100 µm, zum Einsatz kommen können.

Diese Weiterbildung der Erfindung hat nicht nur den Vorteil, dass das gesamte portable medizinische System und insbesondere das mindestens eine Anzeigenelement flexibel und damit einer Umgebung anpassbar sind. Auch hat die Flexibilität des mindestens einen Anzeigeelements einen erheblichen sicherheitsrelevanten Vorteil. Dies hängt insbesondere damit zusammen, dass flexible Displays erheblich höheren mechanischen Schockbelastungen gewachsen sind als übliche starre Displays. So kann es insbesondere bei tragbaren Glukosemessgeräten oder tragbaren Insulinpumpen zu einem Fallenlassen des medizinischen Systems durch den Patienten kommen (insbesondere bei älteren Patienten), was in vielen Fällen zu einer Zerstörung des Anzeigeelements führt. Bei flexiblen Anzeigeelementen ist die Bruchgefahr jedoch erheblich verringert. Auch hier macht sich wiederum der Einsatz von OLEDs positiv bemerkbar, da OLEDs erheblich leichter flexibel herstellbar sind als Flüssigkristallanzeigen, bei welchen eine Flüssigkeit zwischen zwei transparente Substrate eingefüllt wird.

Bei einer weiteren vorteilhaften Ausgestaltung des portablen medizinischen Systems wird eine Optimierungsvorrichtung eingesetzt. Diese Optimierungsvorrichtung soll mindestens einen Helligkeitssensor zur Erfassung einer Helligkeit eines Umgebungslichts aufweisen sowie weiterhin eine Vorrichtung zur automatischen Anpassung, insbesondere eine Optimierung, der Helligkeit und/oder des Kontrasts und/oder des Stromverbrauchs des mindestens einen Anzeigeelements, insbesondere der mindestens einen organischen Leuchtdiodenanzeige. Diese Weiterbildung der Erfindung bietet die Möglichkeit, die Anzeigehelligkeit des mindestens einen Anzeigeelements in Abhängigkeit vom Umgebungslicht automatisch so einzustellen, dass unter jeder Umgebungslichtbedingung optimale Helligkeit (insbesondere kein Überstrahlen im Dunkeln), stets hoher Kontrast sowie stets minimaler Stromverbrauch gegeben sind. Dies ist ein weiterer erheblicher Vorteil der Verwendung von organischen Leuchtdiodenanzeigen gegenüber Flüssigkristallanzeigen, da, wie oben beschrieben, bei Flüssigkristallanzeigen Kontrast und Helligkeit in der Regel nicht gleichzeitig optimierbar sind.

Der Einsatz der Optimierungsvorrichtung bietet insbesondere im Bereich der Diabetes-Medizintechnik besondere Vorteile. Dies hängt teilweise damit zusammen, dass, wie oben beschrieben, als Folge von Diabetes-Erkrankungen häufig Sehstörungen bei den betroffenen Patienten auftreten können. Daher ist es insbesondere hier besonders wichtig, die entsprechenden Anzeigenelemente bezüglich Kontrast und/oder Helligkeit zu optimieren.

Weitere Einzelheiten und Merkmale der Erfindung ergeben sich aus der nachfolgenden Beschreibung des Ausführungsbeispiels in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Im Einzelnen zeigt:

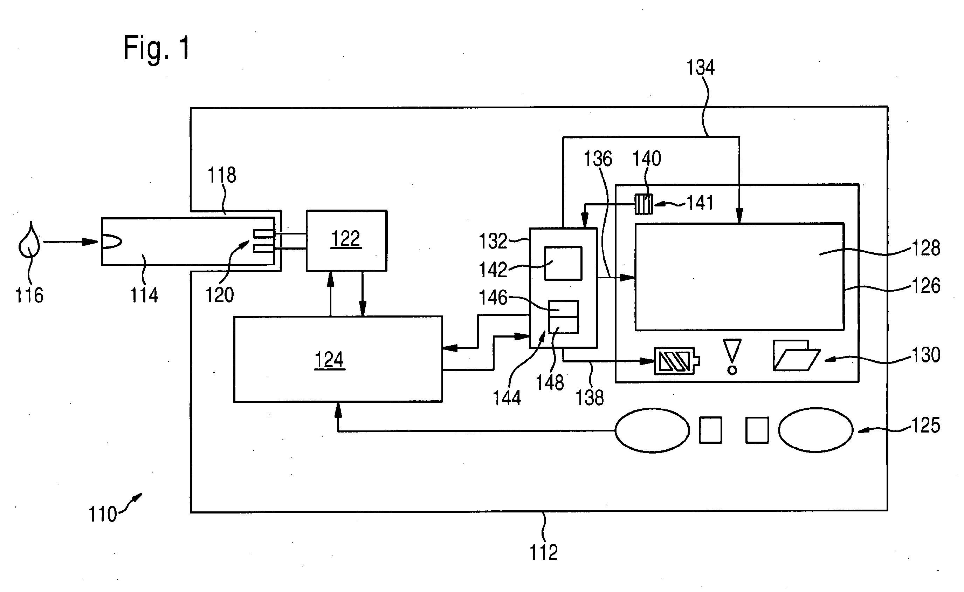

- Figur 1: ein Ausführungsbeispiel eines portablen medizinischen Systems mit einer or- ganischen Leuchtdiodenanzeige.

In Figur 1 ist ein Ausführungsbeispiel eines portablen medizinischen Systems 110 gemäß der Erfindung dargestellt. Das portable medizinische System 110 weist in diesem Ausführungsbeispiel ein Blutglucose-Messgerät 112 auf, welches elektrochemisch mittels eines Teststreifens 114 den Blutglucosegehalt in einem Blutstropfen 116 bestimmen kann. Erfindungsgemäß kann, wie oben beschrieben, anstelle eines Blutglucose-Messgerätes 112 auch eine Insulinpumpe eingesetzt werden.

Das Blutglucose-Messgerät 112 umfasst einen Eingabeschlitz 118, in welchen der mit entsprechenden Elektroden 120 ausgestattete Teststreifen 114 eingeschoben werden kann. Der Teststreifen 114 kann beispielsweise ausgestattet sein wie aus dem Stand der Technik bekannte Teststreifen, beispielsweise die aus US 5,286,362 bekannten Teststreifen. Die Elektroden 120 des Teststreifens 114 werden bei in den Eingabeschlitz 118 eingeführtem Teststreifen 114 von einer Auswertungs- und Messelektronik 122 kontaktiert und es wird eine elektrochemische Bestimmung der Analytkonzentration im Blutstropfen 116 durchgeführt.

Die Auswertungs- und Messelektronik 122 ist verbunden mit einer zentralen Prozessoreinheit 124, welche die Messung durch die Auswertungs- und Messelektronik 122 steuert und Messergebnisse auswertet. Die zentrale Prozessoreinheit 124 kann zusätzlich über verschiedene Speicher verfügen, und es können weitere, nicht dargestellte Datenspeicher vorgesehen sein, insbesondere Speicher mit einer Datenbankfunktion. Auf diese Weise können die mit der Auswertungs- und Messelektronik 122 durchgeführten Messungen nicht nur ausgewertet sondern auch entsprechend gespeichert und dargestellt werden, so dass diese jederzeit für einen Patienten zur Verfügung stehen. Ein Benutzer kann beispielsweise über Bedienelemente 125 die Funktionen der zentralen Prozessoreinheit 124 nutzen und somit eine Messung durchführen oder auch ältere, gespeicherte Messergebnisse darstellen. Derartige Funktionalitäten von Blutglucose-Messgeräten 112 sind aus dem Stand der Technik bekannt.

Weiterhin weist das in Figur 1 dargestellte portable medizinische System 110 ein Anzeigenelement 126 auf. Dieses Anzeigenelement 126 umfasst in diesem Ausführungsbeispiel ein OLED-Passiv-Matrix-Display 128, beispielsweise ein monochromes oder farbiges VGA-Display, also ein Display mit einer Auflösung von 640 x 480 Bildpunkten, oder andere, beispielsweise kleinere, Displays. Durch entsprechende Ansteuerung dieser Bildpunkte können alphanumerische Zeichnungen, Graphiken, Symbole und sogar Videobilder auf dem OLED-Passiv-Matrix-Display 128 dargestellt werden. Alternativ oder zusätzlich könnten auch ein oder mehrere OLED-Aktiv-Matrix-Displays eingesetzt werden.

Weiterhin umfasst das Anzeigenelement 126 eine Symbolleiste 130 mit einzelnen Leuchtsymbolen, beispielsweise einer segmentierten Batteriestandsanzeige, einem Warnsymbol oder einem Datenbanksymbol. Diese Symbolleiste kann beispielsweise wiederum organische Leuchtdioden umfassen, oder es können auch andere Arten von Anzeigeelementen oder Beleuchtungen vorgesehen sein, beispielsweise Beleuchtungen mit anorganischen Leuchtdioden. Auf diese Weise kann sichergestellt werden, dass die Symbolleiste 130 unabhängig von der Funktionsweise des OLED-Passiv-Matrix-Displays 128 funktioniert und auch bei deren Ausfall einsatzbereit ist.

Das Anzeigenelement 126 wird angesteuert von einer Treibereinheit 132, welche sich wiederum aus einzelnen (nicht dargestellten) Untereinheiten zusammensetzen kann. So können beispielsweise für das OLED-Passiv-Matrix-Display 128 und für die Symbolleiste 130 mit ihren einzelnen Symbolen jeweils unterschiedliche Treiber vorgesehen sein. Über Treiberleitungen 134, 136, 138 werden die einzelnen Bestandteile 128, 130 des Anzeigenelements 126 mit entsprechenden Treibersignalen versorgt, beispielsweise Flüssigkristall-Anzeigelemente mit entsprechenden Spannungen oder OLED-Anzeigenelemente mit entsprechenden Strömen. Für das OLED-Passiv-Matrix-Display 128 ist in diesem Ausführungsbeispiel eine Spalten-Treiberleitung 134 und eine Zeilen-Treiberleitung 136 vorgesehen. Auch andere Ausführungen sind denkbar. Insbesondere kann statt einem Passiv-Matrix-Display auch ein Aktiv-Matrix-Display eingesetzt werden.

Entsprechend müssen die Treiberleitungen 134, 136, 138 in Zahl und Ausgestaltung sowie die Treibereinheit 132 an die tatsächlichen Gegebenheiten angepasst werden. Nicht dargestellt in Figur 1 ist zudem eine Energieversorgung, welche beispielsweise in Form von Batterien oder Akkumulatoren vorliegen kann und welche die zentrale Prozessoreinheit 124 und die Treibereinheit 132 entsprechend mit Energie versorgt.

Weiterhin weist das Anzeigenelement 126 in dem Ausführungsbeispiel gemäß Figur 1 eine Optimierungsvorrichtung 141 mit einem Helligkeitssensor 140 auf, welcher beispielsweise eine Photodiode umfassen kann. Dieser Helligkeitssensor ist vorteilhafterweise auf einer gemeinsamen Oberfläche des Anzeigenelements 126 integriert, so dass der Abstand zum OLED-Passiv-Matrix-Display 128 möglichst gering ist. Es können sogar einzelne Bildpunkte im OLED-Passiv-Matrix-Display 128 ausgelassen und durch entsprechende Photosensoren ersetzt werden.

Der Helligkeitssensor 140 ist wiederum verbunden mit der Treibereinheit 132. Alternativ oder zusätzlich kann auch eine Verbindung zur zentralen Prozessoreinheit 124. vorliegen. Auch können zusätzliche elektronische Komponenten zum Auswerten des Helligkeitssensors 140 vorgesehen sein.

Weiterhin ist - in diesem Fall in der Treibereinheit 132 (alternativ oder zusätzlich auch in der zentralen Prozessoreinheit 124) - eine Vorrichtung 142 zur automatischen Anpassung des Anzeigenelements 126 vorgesehen, welche ebenfalls Bestandteil der Optimierungsvorrichtung 141 ist. Diese Vorrichtung 142 zur automatischen Anpassung des Anzeigenelements wertet die Signale des Helligkeitssensor 140 aus und optimiert automatisch die Funktionsweise der Treibereinheit 132 dahingehend, dass das OLED-Passiv-Matrix-Display 128 mit auf die Helligkeitsverhältnisse angepasstem optimalen Kontrast und optimaler Helligkeit bei gleichzeitig minimalem Stromverbrauch arbeitet. Auch die Helligkeit der Symbolleiste 130 (bzw. einzelner Symbole) kann entsprechend optimiert werden. Eine derartige Optimierung bedeutet jedoch nicht notwendigerweise, dass bei "guten" Umgebungslichtverhältnissen (das heißt bei heller Umgebung) die Leuchtdichte des OLED-Passiv-Matrix-Displays 128 verringert wird und bei "schlechten" Umgebungslichtverhältnissen erhöht. Vielmehr ist es so, dass in dunklen Arbeitsumgebungen aufgrund der Adaption des Auges an die dunklen Umgebungslichtverhältnisse häufig geringere Leuchtdichten ausreichend sind. Bei hellen Arbeitsumgebungen hingegen macht sich in vielen Fällen bei OLED-Passiv-Matrix-Displays 128 eine Reflektion des Umgebungslichts an Elektroden des OLED-Passiv-Matrix-Displays 128 negativ bemerkbar, was den Kontrast verringert und somit zur Erhaltung der Ablesbarkeit häufig höhere Leuchtdichten erfordert. All dies kann in einer Optimierung mit einbezogen werden.

Weiterhin umfass der in Figur 1 dargestellten Ausführungsform die Treibereinheit 132 des Blutglucose-Messgeräts 112 eine Überwachungsvorrichtung 144 zur Überwachung der Funktion des Anzeigeelements 126. Diese Überwachungsvorrichtung 144 setzt sich Zusammen aus einer Stromerfassungsvorrichtung 146 und einer Vergleichsvorrichtung 148. Die Stromerfassungsvorrichtung 146 misst (kontinuierlich oder in bestimmten Zeitabständen) den Treiberstrom oder mehrere Treiberströme, welche von der Treibereinheit 132 zu dem OLED-Passiv-Matrix-Display 128 und/oder zur Symbolleiste 130 fließen. Dabei können Ströme durch die einzelnen Treiberleitungen 134, 136, 138 separat erfasst werden, und des können sogar Ströme durch einzelnen Zeilen oder Spalten des OLED-Passiv-Matrix-Displays 128 separat erfasst werden. Die Vergleichsvorrichtung 148 wertet die Ergebnisse der Stromerfassungsvorrichtung 146 aus und vergleicht die gemessenen Ströme mit vorgegebenen Sollwerten. Beispielsweise kann es sich dabei um Sollwerte handeln, welche in einem Datenspeicher, beispielsweise einem Datenspeicher (nicht dargestellt) der zentralen Prozessoreinheit 124, gespeichert sind. Diese Sollwerte können im Betrieb des Anzeigenelements 126 variabel angepasst werden. Beispielsweise können die Sollwerte an die von der Vorrichtung 142 zur automatischen Anpassung des Anzeigenelenients vorgegebenen optimalen Ströme angepasst werden, so dass die Sollwerte von der jeweiligen Umgebungshelligkeit abhängig sind. Auch andere Ausgestaltungen sind möglich.

Neben Sollwerten können auch Toleranzschwellen vorgegeben sein. So kann beispielsweise vorgeben sein, dass bei Unterschreiten gewisser Werte, beispielsweise einer Abweichung um mehr als einen vorgegebenen Betrag von den vorgegebenen Sollwerten oder bei Überschreiten oder Unterschreiten vorgegebener zusätzlicher Schwellen ein Fehler detektiert wird. In diesem Fall kann die Überwachungsvorrichtung 144 beispielsweise eine entsprechende Fehlermeldung an die zentrale Prozessoreinheit 124 liefern, gegebenenfalls sogar eine Fehlemieldung, welche die genaue Art des Fehlers impliziert. Die zentrale Prozessoreinheit 124 kann dann veranlassen, dass eine entsprechende Warnung an einen Benutzer des Blutglucose-Messgeräts 112 ausgegeben wird. Dabei kann es sich beispielsweise um eine akustische Warnung oder auch um die Aktivierung entsprechender Warnsymbole oder Warnhinweise handeln, beispielsweise eines Warnsymbols in der Symbolleiste 130. Auch können die entsprechenden Fehler in der zentralen Prozessoreinheit 124 oder in separaten Datenspeichern gespeichert werden, um dann bei einer Wartung des Blutglucose-Messgeräts 112 von einem Servicetechniker zu Diagnosezwecken ausgelesen zu werden. Auch können neben dem Anzeigenelement 126 separate Anzeigenelemente vorgesehen sein, beispielsweise ein Anzeigenelement, welches lediglich den Wamtext aufweist, dass das Blutglucose-Messgerät 112 gewartet werden muss und nicht mehr verwendet werden darf.

Ebenfalls anhand von Figur 1 soll eine vorteilhafte Weiterbildung des portablen medizinischen Systems 110 erläutert werden. So ist es möglich, das portable medizinische System 110 mit einer Solarzelle auszustatten. Diese Solarzelle kann beispielsweise auf einer Benutzeroberfläche des portablen medizinischen Systems 110 angeordnet sein, beispielsweise neben dem Anzeigenelement 126, oder auch als Bestandteil dieses Anzeigenelements 126. Diese Solarzelle kann dazu dienen, das portable medizinische System zumindest teilweise mit elektrischer Energie zu versorgen. Zusätzlich können weitere Energiespeicher eingesetzt werden, beispielsweise Batterien und/oder Akkumulatoren. Auch eine Aufladung dieser zusätzlichen Energiespeicher durch die von der mindestens einen Solarzelle gelieferte Energie ist denkbar.

Besonders bevorzugt ist es dabei, wenn diese mindestens eine Solarzelle, wie in Figur 1 dargestellt, zumindest teilweise Bauteil-identisch ist mit dem Helligkeitssensor 140. So kann dieser Helligkeitssensor 140 vorteilhafterweise vollständig durch eine entsprechende Solarzelle ersetzt werden. Dann ist vorteilhafterweise das portable medizinische System 110 derart eingerichtet, dass in Abhängigkeit von der von der mindestens einen Solarzelle bereitgestellten elektrischen Energie, beispielsweise in Abhängigkeit von einem Solarstrom, die Helligkeit und/oder der Kontrast des Anzeigeelements 126 eingestellt wird. Dies kann beispielsweise dadurch erfolgen, dass bei starker Sonnenbestrahlung der von der Solarzelle zusätzlich gelieferte Strom genutzt wird, um das Anzeigenelement 126, insbesondere das OLED-Display 128, mit einer größeren Leuchtdichte zu betreiben. Auf diese Weise wird beispielsweise bei direkter Sonneneinstrahlung die Ablesbarkeit des OLED-Displays 128 erhöht. In diesem Fall bildet die Solarzelle einen Teil der Optimierungsvorrichtung 141, welche automatisch die Helligkeit und/oder den Kontrast des Anzeigenelements 126 einstellt. In diesem Fall kann auf eine aufwendige zusätzliche Regelung der Display-Helligkeit oder des Kontrastes verzichtet werden, oder der Aufwand für eine derartige Elektronik kann erheblich reduziert werden, da das "Mess"-Signal der Sonneneinstrahlung unmittelbar in die Stellgröße des Treiberstroms für das Anzeigenelement 126 einfließen kann.

Grundsätzlich lassen sich für die Solarzelle die üblichen aus der Solartechnik bekannten Solarzellensysteme einsetzen. So lassen sich beispielsweise anorganische Solarzellen mit mindestens einem anorganischen Halbleitermaterial einsetzen. Alternativ oder zusätzlich kann die Solarzelle jedoch auch eine organische Solarzelle umfassen, also eine Solarzelle mit mindestens einem organischen Halbleitermaterial. So lassen sich beispielsweise Solarzellen mit Polymeren oder niedermolekularen organischen Materialien als organischen Halbleiter einsetzen, oder auch Hybrid-Bauelemente mit sowohl organischen als auch anorganischen Komponenten. Derartige organische Solarzellen, welche beispielsweise konjugierte Polymere einsetzen, sind beispielsweise aus den Arbeiten von A. Heeger et al. bekannt, für welche im Jahr 2000 der Chemie-Nobelpreis vergeben wurde. Die Verwendung organischer Solarzellen in Kombination mit dem OLED-Display 128 bietet insbesondere den technisch interessanten Vorteil, dass beispielsweise Anzeigenelemente 126 verwendet werden können, welche organische Helligkeitssensoren 140 in Form von organischen Solarzellen, sowie die OLED-Displays 128 und gegebenenfalls die Symbolleiste 130 umfassen, welche vollständig in organischer Halbleitertechnik hergestellt sein können (all organic devices). Auf den Einsatz teurer anorganischer Halbleitertechnik könnte in diesem Fall vorzugsweise vollständig verzichtet werden.

### Bezugszeichenliste

- 110: portables medizinisches System
- 112: Blutglucose-Messgerät
- 114: Teststreifen
- 116: Blutstropfen
- 118: Eingabeschlitz
- 120: Elektroden
- 122: Auswertungs- und Messelektronik
- 124: zentrale Prozessoreinheit
- 125: Bedienelemente
- 126: Anzeigenelement
- 128: OLED-Passiv-Matrix-Display
- 130: Symbolleiste
- 132: Treibereinheit
- 134: Spalten-Treiberleitung
- 136: Zeilen-Treiberleitung
- 138: Treiberleitung
- 140: Helligkeitssensor
- 141: Optimierungsvorrichtung
- 142: Vorrichtung zur automatischen Anpassung des Anzeigenelements
- 144: Überwachungsvorrichtung
- 146: Stromerfassungsvorrichtung
- 148: Vergleichsvorrichtung

## Patentansprüche

1. Portables medizinisches System (110), insbesondere für Analyse- und/oder Medikationszwecke, umfassend mindestens eine medizinische Überwachungsvorrichtung und/oder Analysevorrichtung (112) und/oder Medikationsvorrichtung sowie weiterhin mindestens ein Anzeigenelement (126), wobei das mindestens eine Anzeigenelement (126) mindestens eine organische Leuchtdiodenanzeige (128) umfasst, **dadurch gekennzeichnet, dass** das portable medizinische System (110) eine Überwachungsvorrichtung (144) umfasst, wobei die Überwachungsvorrichtung (144) eine Vorrichtung (146) zur Erfassung eines Treiberstromes der mindestens einen organischen Leuchtdiodenanzeige (128) umfasst, wobei das portable medizinische System (110) weiterhin eine Vorrichtung (148) zum Vergleich des Treiberstroms mit einem vorgegebenen Sollwert umfasst, wobei die Überwachungsvorrichtung (144) mindestens eine Warnvorrichtung (124, 130) zur Erzeugung einer Warnung für einen Benutzer bei Abweichung des Treiberstroms von dem vorgegebenen Sollwert um mehr als einen vorgegebenen Betrag aufweist.

2. Portables medizinisches System (110) gemäß dem vorhergehenden Anspruch, umfassend mindestens ein Analysesystem (112) zum Nachweis mindestens eines Analyten in einer Probe, vorzugsweise einer flüssigen Probe (116), insbesondere zum Nachweis von Blutglucose und/oder Cholesterol und/oder einer Koagulation.

3. Portables medizinisches System (110) gemäß dem vorhergehenden Anspruch, **gekennzeichnet durch** Mittel (114) zum Nachweis des Analyten mittels mindestens eines Reagens.

4. Portables medizinisches System (110) gemäß einem der vorhergehenden Ansprüche, umfassend mindestens eine Medikationspumpe, insbesondere eine Insulinpumpe.

5. Portables medizinisches System (110) gemäß einem der vorhergehenden Ansprüche, **dadurch gekennzeichnet, dass** die mindestens eine organische Leuchtdiodenanzeige (128) mindestens ein Matrix-Display (128), insbesondere ein organisches Passiv-Matrix Display (128), umfasst.

6. Portables medizinisches System (110) gemäß einem der vorhergehenden Ansprüche, **dadurch gekennzeichnet, dass** die mindestens eine organische Leuchtdiodenanzeige (128) mindestens ein flexibles Anzeigenelement (126) aufweist, insbesondere ein Anzeigenelement (126) mit einem transparenten flexiblen Substrat, insbesondere einem flexiblen Glas- und/oder Kunststoffsubstrat.

7. Portables medizinisches System (110) gemäß einem der vorhergehenden Ansprüche, **gekennzeichnet durch** eine Optimierungsvorrichtung (141), umfassend mindestens einen Helligkeitssensor (140) zur Erfassung einer Helligkeit eines Umgebungslichts sowie weiterhin umfassend eine Vorrichtung (142) zur automatischen Anpassung, insbesondere einer Optimierung, mindestens eines der folgenden Parameter des mindestens einen Anzeigenelements (126): einer Helligkeit, eines Kontrasts, eines Stromverbrauchs.

8. Portables medizinisches System (110) gemäß einem der vorhergehenden Ansprüche, **gekennzeichnet durch** mindestens eine Solarzelle zur zumindest teilweisen Versorgung des portablen medizinischen Systems (110) mit elektrischer Energie.

9. Portables medizinisches System (110) gemäß den Ansprüchen 7 und 8, **dadurch gekennzeichnet, dass** die mindestens eine Solarzelle und der mindestens eine Helligkeitssensor (140) zumindest teilweise bauteilidentisch sind.

10. Portables medizinisches System (110) gemäß dem vorhergehenden Anspruch, **dadurch gekennzeichnet, dass** das portable medizinische System (110) eingerichtet ist, in Abhängigkeit von der von der mindestens einen Solarzelle bereitgestellten elektrischen Energie, insbesondere in Abhängigkeit von einem von der mindestens einen Solarzelle bereitgestellten elektrischen Strom, eine Helligkeit und/oder einen Kontrast des mindestens einen Anzeigenelements (126) einzustellen.

11. Portables medizinisches System (110) gemäß einem der drei vorhergehenden Ansprüche, **dadurch gekennzeichnet, dass** die Solarzelle eine anorganische Solarzelle mit mindestens einem anorganischen Halbleitermaterial und/oder eine organische Solarzelle mit mindestens einem organischen Halbleitermaterial umfasst.

## Claims

1. Portable medical system (110), in particular for analysis and/or medication purposes, comprising at least one medical monitoring device and/or analysis device (112) and/or medication device and furthermore at least one display element (126), wherein the at least one display element (126) comprises at least one organic light emitting diode display (128), **characterized in that** the portable medical system (110) comprises a monitoring device (144), wherein the monitoring device (144) comprises a device (146) for recording a drive current of the at least one organic light emitting diode display (128), wherein the portable medical system (110) furthermore comprises a device (148) for comparison of the drive current with a given set value, wherein the monitoring device (144) has at least one warning device (124, 130) for generating a warning for a user if the drive current deviates from the given set value by more than a given amount.

2. Portable medical system (110) according to the preceding claim, comprising at least one analysis system (112) for detection of at least one analyte in a sample, preferably a liquid sample (116), in particular for detection of blood glucose and/or cholesterol and/or a coagulation.

3. Portable medical system (110) according to the preceding claim, **characterized by** means (114) for detection of the analyte by means of at least one reagent.

4. Portable medical system (110) according to one of the preceding claims, comprising at least one medication pump, in particular an insulin pump.

5. Portable medical system (110) according to one of the preceding claims, **characterized in that** the at least one organic light emitting diode display (128) comprises at least one matrix display (128), in particular an organic passive matrix display (128).

6. Portable medical system (110) according to one of the preceding claims, **characterized in that** the at least one organic light emitting diode display (128) has at least one flexible display element (126), in particular a display element (126) with a transparent flexible substrate, in particular a flexible substrate of glass and/or plastic.

7. Portable medical system (110) according to one of the preceding claims, **characterized by** an optimization device (141), comprising at least one brightness sensor (140) for recording a brightness of a surrounding light and furthermore comprising a device (142) for automatic adjustment, in particular an optimization, of at least one of the following parameters of the at least one display element (126): a brightness, a contrast, a power consumption.

8. Portable medical system (110) according to one of the preceding claims, **characterized by** at least one solar cell for at least partially supplying the portable medical system (110) with electrical energy.

9. Portable medical system (110) according to claims 7 and 8, **characterized in that** the at least one solar cell and the at least one brightness sensor (140) are at least partly identical in construction.

10. Portable medical system (110) according to the preceding claim, **characterized in that** the portable medical system (110) is equipped to establish a brightness and/or a contrast of the at least one display element (126) as a function of the electrical energy provided by the at least one solar cell, in particular as a function of an electric current provided by the at least one solar cell.

11. Portable medical system (110) according to one of the three preceding claims, **characterized in that** the solar cell comprises an inorganic solar cell with at least one inorganic semiconductor material and/or an organic solar cell with at least one organic semiconductor material.

## Revendications

1. Système médical portable (110), notamment à des fins d'analyse et/ou de médication, comprenant au moins un dispositif médical de contrôle et/ou d'analyse (112) et/ou un dispositif de médication ainsi qu'en outre au moins un élément d'affichage (126), l'au moins un élément d'affichage (126) comprenant au moins un affichage à diodes luminescentes organiques (128), **caractérisé en ce que** le système médical portable (110) comprend un dispositif de contrôle (144), le dispositif de contrôle (144) comprenant un dispositif (146) d'enregistrement d'un courant d'attaque de l'au moins un affichage à diodes luminescentes organiques (128), le système médical portable (110) comprenant en outre un dispositif (148) destiné à la comparaison du courant d'attaque avec une valeur théorique spécifiée, le dispositif de contrôle (144) présentant au moins un dispositif avertisseur (124, 130) destiné à générer un avertissement pour un utilisateur en cas d'écart du courant d'attaque par rapport à la valeur théorique spécifiée de plus d'un montant spécifié.

2. Système médical portable (110) selon la revendication précédente, comprenant au moins un système d'analyse (112) destiné à détecter au moins un analyte dans un échantillon, de préférence un échantillon liquide (116), notamment pour détecter du sucre dans le sang et/ou du cholestérol et/ou une coagulation.

3. Système médical portable (110) selon la revendication précédente, **caractérisé par** des moyens (114) destinés à détecter l'analyte au moyen au moins d'un réactif.

4. Système médical portable (110) selon l'une quelconque des revendications précédentes, comprenant au moins une pompe de médication, notamment une pompe à insuline.

5. Système médical portable (110) selon l'une quelconque des revendications précédentes, **caractérisé en ce que** l'au moins un affichage à diodes luminescentes organiques (128) comprend au moins un écran à matrice (128), notamment un écran à matrice passif organique (128).

6. Système médical portable (110) selon l'une quelconque des revendications précédentes, **caractérisé en ce que** l'au moins un affichage à diodes luminescentes organiques (128) présente au moins un élément d'affichage flexible (126), notamment un élément d'affichage (126) comprenant un substrat flexible transparent, notamment un substrat en verre et/ou en matière plastique flexible.

7. Système médical portable (110) selon l'une quelconque des revendications précédentes, **caractérisé par** un dispositif d'optimisation (141), comprenant au moins un capteur de luminosité (140) destiné à enregistrer une luminosité d'une lumière environnante et comprenant en outre un dispositif (142) pour l'adaptation automatique, notamment une optimisation, au moins de l'un des paramètres suivants de l'au moins un élément d'affichage (126): une luminosité, un contraste, une consommation de courant.

8. Système médical portable (110) selon l'une quelconque des revendications précédentes, **caractérisé par** au moins une cellule solaire pour l'alimentation, au moins partielle, en énergie électrique du système médical portable (110).

9. Système médical portable (110) selon les revendications 7 et 8, **caractérisé en ce que** l'au moins une cellule solaire et l'au moins un capteur de luminosité (140) sont de construction identique au moins en partie.

10. Système médical portable (110) selon la revendication précédente, **caractérisé en ce que** le système médical portable (110) est configuré pour régler une luminosité et/ou un contraste de l'au moins un élément d'affichage (126) en fonction de l'énergie électrique fournie par l'au moins une cellule solaire, notamment en fonction d'un courant électrique fourni par l'au moins une cellule solaire.

11. Système médical portable (110) selon l'une quelconque des trois revendications précédentes, **caractérisé en ce que** la cellule solaire comprend une cellule solaire inorganique comprenant au moins un matériau de semi-conducteur inorganique et/ou une cellule solaire organique comprenant au moins un matériau de semi-conducteur organique.
